# EUROPÄISCHE PATENTSCHRIFT

(11) **EP 0 469 445 B1**
(45) Veröffentlichungstag und Bekanntmachung des Hinweises auf die Patenterteilung: **15.05.1996**
(21) Anmeldenummer: 91112389.1
(22) Anmeldetag: 24.07.1991
(51) Int. Cl.: G01N 33/52, G01N 33/543, G01N 33/78

(54) **Analyseelement und Verfahren zu seiner Herstellung**
Analytical element and process for its production
Dispositif d'épreuve et procédé de fabrication

(30) Priorität: 02.08.1990 DE 4024544
(43) Veröffentlichungstag der Anmeldung: 05.02.1992
(73) Patentinhaber: BOEHRINGER MANNHEIM GMBH, D-68298 Mannheim (DE)
(72) Erfinder: Deeg, Rolf, Dr. rer. nat., W-8139 Bernried (DE); Maurer, Eberhard, Dr. rer. nat., W-8120 Weilheim (DE); Klose, Sigmar, Dr. phil., W-8131 Berg 2 (DE); Köpfer, Bernhard, W-8132 Tutzing (DE); Babiel, Reiner, Dr. rer. nat., W-8121 Eberfing (DE)
(74) Vertreter: Pfeifer, Hans-Peter, Dr., Dr. H.-P. Pfeifer Dr. P. Jany, Patentanwälte

(56) Entgegenhaltungen:
- EP-A- 0 061 167
- EP-A- 0 192 428
- EP-A- 0 212 642
- EP-A- 0 299 428
- WO-A-84/04171
- DE-A- 3 346 795
- FR-A- 2 355 290

## Beschreibung

Die Erfindung betrifft ein Analyseelement nach dem Oberbegriff des Anspruchs 1.

Analytische Untersuchungen an flüssigen Proben, insbesondere Körperflüssigkeiten wie Blut oder Urin, werden vielfach mit Hilfe von Analyseelementen durchgeführt, die auch als Testträger und in der angelsächsischen Literatur vielfach als "solid state analysis elements" bezeichnet werden. Sie werden in verschiedenen äußeren Formen, insbesondere als langgestreckte Teststreifen und als quadratische Plättchen angeboten. In jedem Fall weisen sie eine oder mehrere Testschichten auf, welche die für die Analyse erforderlichen Reagenzien enthalten. Die Testschichten werden mit der Probenflüssigkeit kontaktiert, und die Reaktion des Analyten mit den Reagenzien führt zu einem physikalisch meßbaren Nachweissignal, insbesondere einer Farbänderung, die visuell oder photometrisch vermessen wird. Beispiele anderer bekannten Nachweissignale sind die optische Fluoreszenz, Lumineszenz, sowie Spannungs- oder Stromsignale bei elektrochemischen Analyseelementen.

Analyseelemente, die mit einer spezifischen Bindungsreaktion zweier bioaffiner Bindungspartner arbeiten, sind in neuerer Zeit von besonderer Bedeutung. Spezifische Bindungsreaktionen in diesem Sinne sind insbesondere immunologische Interaktionen, also Wechselwirkungen zwischen Antigenen bzw. Haptenen einerseits und Antikörpern andererseits. Es können jedoch auch andere spezifische bioaffine Wechselwirkungen verwendet werden, wie Lectin-Zucker, eine Wirkstoff-Receptor-Wechselwirkung, die spezifische Bindung zwischen Biotin und Streptavidin oder bestimmte Enzym-Substratbindungen, wie z.B. Inhibitoren oder Suicide-Substrate.

Die Reagenzien sind überwiegend in die Testschichten inkorporiert, wobei im Regelfall entweder eine poröse Trägermatrix (z.B. aus Papier oder Kunststoff) mit Reagenz imprägniert wird oder in einem schichtbildenden Verfahren ein Reagenzfilm erzeugt wird, welcher die Reagenzien in einem Filmbildner gelöst oder dispergiert enthält. Bei der Herstellung von Analyseelementen müssen in der Regel verschiedene, miteinander unverträgliche Reagenzien räumlich getrennt untergebracht werden. Dies geschieht üblicherweise durch Zusammenfügen (z.B. Verschweißen, Verkleben) von einzeln vorgefertigten Reagenzträgerelementen. Diese Verfahren sind sehr aufwendig, verursachen häufig Produktionsfehler und ermöglichen nur eine begrenzte Miniaturisierung.

In der WO 84/04171 sind Analyseelemente, insbesondere für Schwangerschaftstests, beschrieben, bei denen Bindungspartner bioaffiner Bindungsreaktionen, insbesondere Antikörper oder Antigene mit Hilfe einer beliebigen Drucktechnik auf der Testfläche appliziert werden. Um die dabei erwarteten besonderen Schwierigkeiten, insbesondere wegen der Migration von Reagenzien, zu vermeiden, wird die Testfläche großflächig mit Avidin beschichtet. Hierauf wird ein biotinylierter Antikörper derartig aufgedruckt, daß das Druckmuster stets nur eine Teilfläche der Avidin-beschichteten Oberfläche bedeckt. Durch die unmittelbare Bindung des biotinylierten Antikörpers an das Avidin soll das Problem der Reagenzienmigration eliminiert werden.

In neuerer Zeit ist vorgeschlagen worden, bei der Herstellung von Analyseelementen die ursprünglich für Computerdrucker (Tintenstrahldrucker) entwickelte Ink-Jet-Technologie zu verwenden (EP-A-119573 und EP-A-268237). Beide Schriften enthalten nähere Erläuterungen zum vorbekannten Stand der Technik, insbesondere auch zu der Ink-Jet-Technologie, auf die hier Bezug genommen wird.

Die Ink-Jet-Technik zeichnet sich dadurch aus, daß sehr kleine Quanten (Teilmengen) einer Flüssigkeit als Tropfen in hoher Präzision auf eine Tragschicht appliziert werden können. Die Präzision bezieht sich dabei sowohl auf die exakte Positionierung des von dem Reagenztropfen erzeugten Punktes (im folgenden als "Dot" bezeichnet) auf der Reagenzfläche als auch auf das Reagenzvolumen. Die Tropfen können mit hoher Frequenz hintereinander ausgestoßen werden.

Reagenzmuster, die mit einem Ink-Jet-Verfahren erzeugt werden, unterscheiden sich eindeutig von den mit anderen Drucktechniken erhältlichen Mustern. Insbesondere sind auf keine andere Weise vergleichbar feine Reagenzpunkte in ähnlicher Gleichmäßigkeit zu erzeugen.

Eine besondere Variante der Ink-Jet-Technik, die auch für die Erfindung besonders geeignet ist, ist die "Drop-on-demand-Technik", bei der zu einem beliebigen Zeitpunkt individuelle Flüssigkeitstropfen erzeugt und auf eine Tragschicht appliziert werden können. Im Zusammenhang mit der Dosierung biochemischer Analyseflüssigkeiten, insbesondere Reagenzien, wurde bisher lediglich die in den genannten Schriften beschriebene Technik angewandt, bei der jeweils das Volumen einer Düsenkammer komprimiert wird, wenn ein Tropfen ausgestoßen werden soll. Insbesondere wird hierbei eine piezoelektrische Veränderung des Düsenkammervolumens angewendet. In der gleichzeitig eingereichten europäischen Patentanmeldung mit der Publikationsnummer 0 469 444 wird die Verwendung der Bubble-Jet-Technologie für das Applizieren von Reagenzflüssigkeiten auf eine Reagenzfläche beschrieben, die ebenfalls für die vorliegende Erfindung geeignet ist. Auch auf diese Anmeldung wird hier Bezug genommen. Der Begriff Ink-Jet wird nachfolgend so verstanden, daß er beide genannten Verfahrensweisen umfaßt.

Die Ink-Jet-Technik ermöglicht es, Reagenzien in hoher Präzision und Gleichmäßigkeit als Reagenzschicht von außerordentlich geringer Schichtstärke auf einen Reagenzbereich eines Analyseelementes zu applizieren. In den genannten Schriften wird auch die Möglichkeit angesprochen, daß das Reagenz auf der Tragschicht in einem bestimmten Muster appliziert wird, um z.B. einen unmittelbaren Vergleich zwischen einem mit Reagenz beschichtetem Teilbereich und einem reagenzfreien Teilbereich zu ermöglichen oder um das Analyseresultat deutlicher sichtbar zu machen, weil die Farbbildung beispielsweise in Form eines Plus- oder Minuszeichens erscheint.

Aus der DE-A-27 27 347 und der DE-C- 27 29 233 ist eine alternierende Anordnung von Flächenelementen (insbesondere Punkten oder Flecken) von verschiedenen Reagenzien bekannt, die mit einer Siebdrucktechnik aufgebracht sind. Dieses Verfahren ist jedoch sehr aufwendig. Außerdem ist keine exakte Dosierung der aufgetragenen Reagenzmengen möglich. Die Flächenelemente sind verhältnismäßig groß und können nur begrenzt miniaturisiert werden. Viele Reagenzien können nicht zu siebdruckfähigen Pasten verarbeitet werden ohne sie zu schädigen oder ihre Eigenschaften zu verändern. Dieses seit langem bekannte Verfahren hat deswegen keine praktische Bedeutung erlangt.

Ein weiteres Beispiel für die alternierende Anordnung unterschiedlicher Reagenzien auf der Oberfläche eines Analyseelementes ist in der FR-A-2 355 290 beschrieben. Als geeignet für die drucktechnische Applikation des vorzugsweise punkt- oder streifenförmigen Reagenzmusters wird jede Art von reproduzierbaren graphischen Techniken bezeichnet. Dabei wird auch die Ink-Jet-Technik erwähnt, in dem einzigen Beispiel der Druckschrift wird jedoch mit einer konventionellen Drucktechnik gearbeitet.

Gemäß der Erfindung ist ein Analyseelement der eingangs bezeichneten Art durch die Merkmale des Anspruchs 1 gekennzeichnet. Der Abstand der äußeren Begrenzungen der Kompartimente verschiedener Sätze liegt im Mittel typischerweise unter 1 mm, vorzugsweise unter 0,5 mm. Die Kombination der erfindungsgemäßen Maßnahmen wird nachfolgend auch als "Mikrokompartimentierung" bezeichnet.

Der Begriff "Kompartiment" (englisch: compartment) bezeichnet dabei einen abgegrenzten Teilbereich. Ein Kompartiment kann aus einem Dot oder aus mehreren einander überlappenden Dots bestehen. Die Kompartimente, welche verschiedene Reagenzien enthalten (und deswegen verschiedenen Sätzen von Kompartimenten in dem Reagenzbereich angehören), sind zumindest teilweise dadurch räumlich voneinander getrennt, daß sie (wenn auch nicht notwendigerweise in der gleichen Ebene) nebeneinander in dem Reagenzbereich angeordnet sind, wobei die Kompartimente mit verschiedenen Reagenzien alternieren, d.h. wechselweise Kompartimente aus verschiedenen Sätzen benachbart sind. Aus praktischen Gründen ist ein regelmäßiges Alternieren zweckmäßig, bei dem also beispielsweise die Kompartimente von drei Sätzen A, B und C in einem sich zyklisch wiederholenden Muster A,B,C,A,B,C,A,B ... vorliegen. In Ausnahmefällen kann aber auch ein alternierendes Muster ohne zyklische Wiederholung zweckmäßig sein.

Die Kompartimente können zum Teil auch übereinander angeordnet sein. Dabei ist eine räumliche Trennung der Kompartimente in Richtung senkrecht zu ihrer Flächenausdehnung (vertikale Kompartimentierung) möglich, wenn eine isolierende Zwischenschicht aus einer löslichen inerten Isolationssubstanz (z.B. einem inerten Protein oder Filmbildner) appliziert wird.

Bei dem erfindungsgemäßen Verfahren liegt das Volumen eines von einem Ink-Jet-Düsenkopf ausgestoßenen Quantums Reagenzflüssigkeit typischerweise zwischen 20 und 2000 Pikoliter, bevorzugt zwischen 100 und 800 Pikoliter. Die Fläche des von einem solchen Quantum auf der Tragschicht erzeugten Dot ist stark von den Eigenschaften der Reagenzflüssigkeit und der Tragschicht abhängig. Sie liegt etwa zwischen 500 µm und 0,2 mm, bevorzugt zwischen 3000 µm und 0,1 mm. Die Reagenzflüssigkeitsquanten werden typischerweise mit einer Frequenz von mehr als 1000 sec⁻¹, bevorzugt zwischen 2000 und 20.000 sec⁻¹ ausgestoßen.

Die Kompartimente erfindungsgemäßer Analyseelemente enthalten sowohl eluierbare als auch an die Tragschicht festphasengebundene Reagenzien. Kompartimente mit eluierbaren Reagenzien werden als "eluierbare Kompartimente" und Kompartimente mit festphasengebundenen Reagenzien als "fixierte Kompartimente" bezeichnet. Dabei werden erfindungsgemäß unter anderem folgende Vorteile erzielt.

Die Reagenzien eluierbarer Kompartimente werden schnell von der Probenflüssigkeit gelöst und miteinander gemischt. Durch Zugabe von die Löslichkeit modifizierenden Bestandteilen zu der Reagenzflüssigkeit kann das Löslichkeitsverhalten der einzelnen Reagenzien in den Kompartimenten unterschiedlicher Sätze modifiziert werden, um einen bestimmten Reaktionsablauf zu ermöglichen. Auch durch Variation der Schichtdicke verschiedener Kompartimente kann das Auflösungsverhalten so beeinflußt werden, daß eine flexible Anpassung an den jeweiligen Reaktionsablauf möglich ist. Beispiele von Reagenzien, die überwiegend in eluierbarer Form verwendet werden, sind Enzyme, Substrate, Coenzyme und Nachweiskomponenten, insbesondere Farbbildungsreagenzien.

Festphasengebundene Reagenzien können sowohl adsorptiv, als auch kovalent gebunden werden. Bei der adsorptiven Bindung ist vorteilhaft, daß (im Gegensatz zur Siebdrucktechnik) Beschichtungslösungen auf wässriger Basis verwendet werden können, die keine die adsorptive Bindung störenden hydrophoben Zusatzstoffe enthalten. Reagenzien, die auf der Tragschicht festphasengebunden werden sollen, können präzise lokalisiert werden, wobei insbesondere die Diffusionsstrecken zu anderen (festphasengebundenen oder eluierbaren) Reagenzien in anderen Sätzen von Kompartimenten den Erfordernissen des Einzelfalls entsprechend festgelegt werden können.

Allgemein ermöglicht die Erfindung sehr kurze Diffusionsstrecken zwischen den in verschiedenen Sätzen von Kompartimenten enthaltenen Reagenzien und damit verhältnismäßig kurze Reaktionszeiten und gute Durchmischung der Reagenzien ohne besondere zusätzliche Maßnahmen.

Im Rahmen der vorliegenden Erfindung wurde festgestellt, daß die mit der Ink-Jet-Technologie mögliche Mikrokompartimentierung völlig neue Testabläufe ermöglicht.

Wie erwähnt bezieht sich die Erfindung auf Bestimmungsmethoden, die auf der spezifischen Bindungsfähigkeit zweier bioaffiner Bindungspartner basieren. Mindestens ein Satz von Kompartimenten enthält einen ersten Bindungspartner, der mit einem zweiten Bindungspartner, welcher in der Probenflüssigkeit enthalten oder ein Reagenz sein kann, spezifisch bindungsfähig und an der Tragschicht fixiert ist.

Bei der Herstellung fixierter Kompartimente ist es vielfach vorteilhaft, wenn der zu fixierende Bindungspartner in einer Konzentration (pro Flächeneinheit) appliziert wird, welche geringer als die Bindungskapazität (pro Flächeneinheit) der Oberfläche, an die er fixiert wird, ist. Dadurch können die sonst bei der Trägerfixierung von Reagenzien erforderlichen weiteren Prozeßschritte, insbesondere das Abwaschen des Überschusses, entfallen.

Die Erfindung wird im folgenden anhand von in den Figuren schematisch dargestellten Ausführungsbeispielen näher erläutert, es zeigen:
- Fig. 1: Eine Aufsicht auf einen Teil eines erfindungsgemäßen Analyseelementes,
- Fig. 2: eine perspektivische Prinzipdarstellung des Reagenzbereiches eines immunologischen Analyseelementes,
- Fig. 3: eine alternative Ausführungsform des Reagenzbereiches eines immunologischen Analyseelementes.

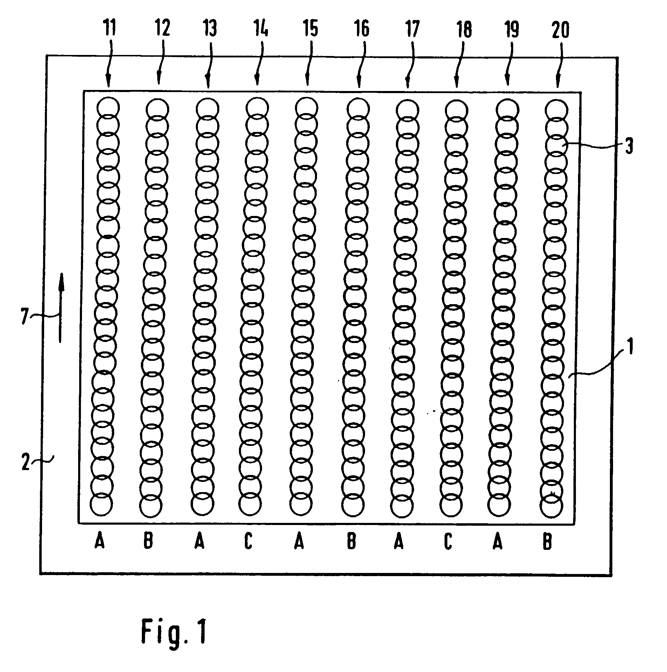

Der in Figur 1 dargestellte Reagenzbereich 1 enthält zehn reihenförmige Reagenzkompartimente 11 bis 20, welche nebeneinander auf eine Tragschicht 2 appliziert sind. Jedes der Kompartimente besteht aus einer Vielzahl von Dots 3, die mit einem Ink-Jet-Druckkopf auf den Reagenzbereich 1 aufgebracht sind. Dabei wird vorzugsweise wie folgt vorgegangen:

Als Tragschicht 2 wird eine Kunststoffolie (insbesondere aus Polystyrol) verwendet, die zuvor unter standardisierten Bedingungen mit Gamma-Strahlen bestrahlt wurde (EP-B1-0 061 167). Danach wird sie unter einem relativ zu der Tragschicht 2 präzise beweglichen Ink-Jet-Druckkopf positioniert und mit den Dots 3 bedruckt.

Um dabei eine gleichmäßige, flächendeckende Applikation des Reagenz jeweils innerhalb der Kompartimente 11 bis 20 zu erzielen, wird eine spezielle Technik angewandt. Von den Dots innerhalb eines Kompartiments wird in einem ersten Verfahrensschritt nur jeder zweite Dot eines Kompartiments appliziert, so daß die in diesem Verfahrensschritt aufgebrachten Reagenzflüssigkeitsquanten räumlich getrennte Dots bilden. Nach Trocknung des Reagenzes (ca.60sec bei Raumtemperatur) werden in einem zweiten zeitlich getrennten Schritt Reagenzflüssigkeitsquanten auf die Zwischenräume zwischen den im ersten Schritt erzeugten Dots derartig appliziert, daß eine durchgehendes Kompartiment gebildet wird. Diese Vorgehensweise ist insbesondere dann vorteilhaft, wenn eine wässrige Reagenzflüssigkeit auf einer verhältnismäßig hydrophoben Oberfläche der Tragschicht 2 appliziert werden muß. Vielfach ist es aber auch möglich, durch Beeinflussung der Oberflächeneigenschaften des Tragschichtmaterials oder durch Modifikation der Zusammensetzung der Reagenzflüssigkeit die Notwendigkeit der beschriebenen zweistufigen Verfahrensführung zu vermeiden.

Die Kompartimente 11 bis 20 sind im dargestellten Fall in ihrer äußeren Form und in ihrem physischen Aufbau gleich (was bevorzugt, aber nicht notwendig ist), unterscheiden sich jedoch hinsichtlich ihrer chemischen Zusammensetzung. Sie lassen sich in Sätze einteilen, die in Fig. 1 mit den Buchstaben A, B und C bezeichnet sind. Die Kompartimente innerhalb eines Satzes enthalten die gleiche Reagenzzusammensetzung, während sich die Kompartimente verschiedener Sätze hinsichtlich ihrer Reagenzzusammensetzung unterscheiden. Dabei sind die Kompartimente unterschiedlicher Sätze derartig alternierend angeordnet, daß die verschiedene Reagenzien enthaltenden Kompartimente eng benachbart aber räumlich getrennt sind.

Im dargestellten Fall ist jedes zweite Kompartiment dem Satz A zuzuordnen. Die Anzahl der Kompartimente der Sätze B und C ist nur jeweils halb so groß und sie sind abwechselnd in den Lücken der Kompartimente des Satzes A plaziert.

Die Abmessungen und Abstände der Kompartimente sind außerordentlich klein. In einem praktisch erprobten Fall beträgt der Dotabstand innerhalb eines Kompartiments nur etwa 0,14 mm, der Abstand der Kompartimente von Mitte zu Mitte lag in diesem Beispielsfall bei etwa 0,26 mm, der Abstand der Begrenzungen der Kompartimente im Mittel unter 0,15 mm.

Die Kompartimente verschiedener Sätze können vorteilhaft in einen Arbeitsgang mit Hilfe eines Mehrkanaldruckkopfes oder eines mit mehreren Druckköpfen ausgestatteten Druckers erzeugt werden. Solche nach dem Ink-Jet-Verfahren arbeitenden Druckertypen sind für den Farbdruck entwickelt worden. Um das in Fig. 1 dargestellte Kompartimentmuster zu erzeugen kann beispielsweise so vorgegangen werden, daß linear angeordnete Düsen eines Mehrkanaldruckkopfes für die verschiedenen Kompartimente eingesetzt werden und der Druckkopf relativ zu der Tragschicht 2 in Richtung senkrecht zu der linearen Düsenanordnung über die Tragschicht 2 bewegt wird (Pfeil 7). Dadurch wird eine zugleich präzise und wirtschaftliche Herstellung der erfindungsgemäßen Analyseelemente möglich.

Die Relativbewegung zwischen Druckkopf und Tragschicht läßt sich mit den für Tintenstrahldrucker gebräuchlichen Konstruktionen realisieren. Im Rahmen der Erfindung ist es vorteilhaft, den Druckkopf unidirektional zu betreiben, um eine besonders präzise positionierung der Dots zu ermöglichen.

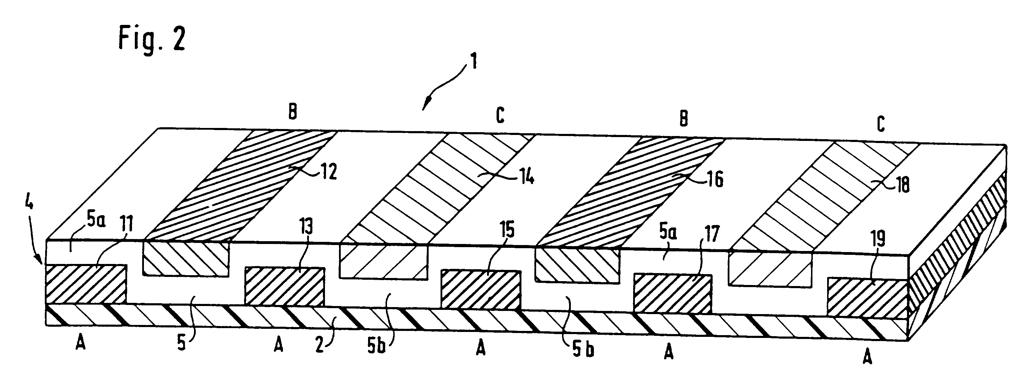

Figur 2 zeigt in einer stark schematisierten prinzipdarstellung den Schichtaufbau im Reagenzbereich eines Analyseelementes für immunologische Bestimmungen.

Auf der Tragschicht 2 sind wiederum drei Sätze von Kompartimenten A (Kompartimente 11, 13, 15, 17 und 19), B (Kompartimente 12 und 16) und C (Kompartimente 14 und 18) dargestellt. In der dreidimensionalen Darstellung ist eine weitere bevorzugte Maßnahme zu erkennen, nämlich daß die einzelnen Kompartimente voneinander durch eine Trennschicht 5 getrennt sind, welche eine inerte wasserlösliche Substanz, insbesondere ein wasserlösliches Eiweiß wie Rinderserumalbumin enthält. Der Auftrag der Trennschicht erfolgt in der Weise, daß nach der Applikation der zu dem Satz A gehörenden Kompartimente das RSA in mehrfachen Durchgängen aus einem Druckkopf aufgetragen wird, wobei nicht nur die auf die Kompartimente 11 bis 20 gerichteten Düsen, sondern auch dazwischenliegende Düsen des Druckkopfes aktiviert sind, um die durchgehende RSA-Schicht zu bilden.

Die Kompartimente 11 bis 19 und die RSA-Trennschicht 5 bilden insgesamt eine Reagenzschicht 4 auf der Tragschicht 2. Die Form der Kompartimente ist in der Praxis nicht so gleichmäßig und rechteckig, wie in Fig. 2 dargestellt. Charakteristisch für die Erfindung ist jedoch, daß die Kompartimente räumlich getrennt voneinander sind, wobei die zu verschiedenen Sätzen gehörenden Kompartimente zumindest teilweise in Richtung der Oberfläche der Reagenzschicht 4 nebeneinander auf der Tragschicht 2 angeordnet sind (Horizontale Kompartimentierung).

In einer bevorzugten Ausführungsform enthalten die Kompartimente des Satzes A einen trägerfixierten ersten Bindungspartner Bp(b) (binding partner, bound). Dabei ist es vorteilhaft, wenn die den ersten Bindungspartner enthaltenden Kompartimente mit einer Schutzschicht 5a aus einer inerten Schutzsubstanz bedeckt sind. Geeignet ist insbesondere ein lösliches, mit den übrigen Testbestandteilen nicht reagierendes Protein. Häufig wird es mit einer Zukkerverbindung gemischt. Durch die Schutzschicht wird die erforderliche Lagerstabilität des trägerfixierten Bindungspartners Bp(b) gewährleistet.

Mindestens ein weiterer Satz von Kompartimenten B oder C enthält einen mit Bp(b) spezifisch bindungsfähigen zweiten freien Bindungspartner Bp(f) (binding partner, free), welcher frei ist, also von der Probenflüssigkeit ohne weiteres gelöst werden kann. Bp(f) ist vorzugsweise nach einem in der Immunologie üblichen Verfahren (beispielsweise durch Konjugation mit einer Markierungssubstanz M, z.B. einem Enzym oder einem Fluoreszenzmarker) markiert. In diesem Fall, aber auch bei anderen eluierbaren Kompartimenten, ist es vorteilhaft, wenn unter den Kompartimenten 12,16 eine die Tragschicht 2 bedeckende Blockierungsschicht schicht 5b angeordnet ist. Die Blockierungsschicht 5b basiert zweckmäßigerweise ebenfalls auf einem löslichen inerten Protein und dient dazu, unspezifische Bindungen des eluierbaren Reagenz (hier Bp(f)) an die Tragschicht 2 zu verhindern und damit die Eluierbarkeit des Bp(f) zu verbessern.

Bei der in Figur 2 dargestellten Ausführungsform gehen die Schutzschichten 5a, welche die Kompartimente 11,13,15,17 und 19 bedecken und die Blockierungsschichten 5b, welche unterhalb der Kompartimente 12,14,16 und 18 angeordnet sind, ineinander über und bilden insgesamt die Trennschicht 5. Dies ist besonders vorteilhaft, jedoch nicht notwendig. Es könnten auch getrennte Schutz- bzw. Blockierungsschichten vorgesehen sein.

Nähere Einzelheiten hängen von dem immunologischen Testprinzip ab, nach welchem das Analyseelement arbeitet. Ist beispielsweise der Analyt ein Antigen Ag(s) (antigen, sample), so kann der Bp(f) bei Verwendung eines kompetitiven Testprinzips ein zu dem Ag(s) analoges Antigen Ag(f) sein. Der Bp(b) ist in diesem Fall ein mit dem Ag(f) spezifisch bindungsfähiger Antikörper Ab(b) (antibody, bound). Der Analyseablauf basiert dabei darauf, daß das Ag(s) mit dem Ag(f) um Bindungsstellen an dem Ab(b) konkurriert, wobei die Menge des an das Ab(b) gebundenen Ag(f), welche aufgrund der Markierung des Ag(f) nachweisbar ist, ein Maß für die Konzentration des Analyten ist. Die Konzentration des Ag(f) kann über die Markierung vorzugsweise im gebundenen Zustand, grundsätzlich jedoch auch in der freien Phase bestimmt werden.

Gemäß einem anderen immunologischen Reaktionsprinzip ("Ein-Schritt-Sandwich") können die Kompartimente B oder C (wiederum zur Bestimmung eines Antigens Ag(s)) einen mit dem Ag(s) spezifisch bindungsfähigen Antikörper Ab(f) enthalten. In diesem Fall enthält der Kompartiment-Satz A einen trägerfixierten Antikörper Ab(b), welcher zu dem Ag(s) über ein zweites Epitop spezifisch bindungsfähig ist. In diesem Fall basiert das Testverfahren darauf, daß das Ag(s) eine Bindung zwischen dem Ab(b) und dem Ab(f) vermittelt.

Im Fall der Bestimmung eines Antikörpers statt eines Antigens sind jeweils die gebundenen bzw. freien immunologischen Reaktionsbestandteile in den Kompartimenten auszutauschen (Antigen gegen Antikörper und umgekehrt).

Diese und andere für die Erfindung geeignete immunologische Testprinzipien sind seit langem bekannt. Verwiesen sei beispielsweise auf das US-Patent 4 861 711 und zahlreiche andere Publikationen, in denen die Anwendung heterogener immunologischer Reaktionen auf Analyseelemente in verschiedenen Varianten beschrieben wird.

Tabelle 1 zeigt die Bestückung der Kompartiment-Sätze für die vorgenannten und einige weitere Reaktionsprinzipien:

**Tabelle 1:**

| | Bp(s) | Bp(b) in A | Bp(f) in B und/oder C |
|---|---|---|---|
| Sandwich | Ag | Ab | Ab-M |
| | Ab | Ag | Ag-M |
| kompetitiv | Ag | Ab | Ag-M |
| | Ab | Ag | Ab-M |

Gegenüber den bekannten immunchemischen Analyseelementen erreicht die Erfindung durch Anwendung der Ink-Jet-Technik in Form von alternierend und räumlich getrennt aber eng beieinander angeordneten Kompartimenten unterschiedlicher immunologischer Reaktionsbestandteile eine bedeutende Vereinfachung der Herstellung und des Aufbaus eines immunologischen Analyseelementes.

Es zeigt sich, daß der in einem ersten Satz von Kompartimenten enthaltene lösliche Bindungspartner von der Probe sehr schnell gelöst wird, so daß die Reaktion dieses Bindungspartners mit dem Analyten unmittelbar nach dem Probenkontakt beginnt. Zugleich steht die gesamte Probe in Kontakt zu dem trägerfixierten Bindungspartner. Die Mikrokompartimentierung der Reagenzien ermöglicht einen schnellen und homogenen Ablauf der jeweiligen Bindungsreaktionen mit einer sehr geringen Probe- und Reagenzmenge und hoher Reaktionsgeschwindigkeit. Dabei läuft zunächst bevorzugt die Reaktion mit dem freien Bindungspartner ab, während die Reaktion mit dem trägerfixierten Bindungspartner als heterogene Reaktion wesentlich langsamer abläuft und praktisch in nennenswertem Umfang erst nach Ablauf der Bindungsreaktion des Bp(f) einsetzt.

Gemäß einer bevorzugten Ausführungsform der Erfindung sind auf die Reagenzfläche eines Analyseelementes mindestens drei verschiedene Sätze von Kompartimenten appliziert, nämlich ein erster trägerfixierter Bindungspartner Bp(b) in dem Satz A, ein zweiter mit diesem spezifisch bindungsfähiger freier Bindungspartner Bp(f)1 in dem Satz B und ein dritter, ebenfalls freier, jedoch zusätzlich eine Markierung aufweisender Bindungspartner Bp(f)2 in dem Satz C. Dabei ist der zweite Bindungspartner Bp(f)1 sowohl mit dem ersten Bindungspartner Bp(b) als auch mit dem dritten Bindungspartner Bp(f)2 mit unterschiedlicher Spezifität bindungsfähig. Vorzugsweise ist der erste Bindungspartner Bp(b) für verschiedene Analyseelemente gleich, während die beiden freien Bindungspartner je nach dem zu bestimmenden Analyten (Parameter) und dem gewählten Verfahren ausgewählt sind. Bevorzugt enthält der erste Bindungspartner Streptavidin (SA) oder Avidin und der zweite Bindungspartner Biotin (B). Das Biotin wird mit einem Antigen oder Antikörper je nach der Testführung konjugiert zu Ag-B bzw. Ab-B.

Tabelle 2 zeigt die Bestückung der Kompartiment-Sätze für zwei verschiedene Testprinzipien, wobei jeweils davon ausgegangen wird, daß ein Antigen in der Probe bestimmt werden soll. Im Falle der Bestimmung eines Antikörpers sind jeweils Antigen und Antikörper zu vertauschen.

**Tabelle 2:**

| Zur Bestimmung eines Ag | | | |
|---|---|---|---|
| | Bp(b) | Bp(f)1 | Bp(f)2 |
| kompetitiv (a) | SA | B-Ag | Ab-M |
| (b) | SA | B-Ab | Ag-M |
| Sandwich | SA | B-Ab | Ab-M |

Bei jedem dieser Prinzipien bindet nach Auflösung der freien Bindungspartner das Bp(f)1 über die Avidin-Biotin-Bindung an das Bp(b).

Bei dem kompetitiven Test des Typs (a) wird die Bindung des Ab-M an das B-Ag durch die Kompetition mit dem Probenantigen Ag(s) bestimmt. Bei dem kompetitiven Test des Typs (b) konkurriert das Ag(s) mit dem Ag-M um Bindungsstellen an dem Antikörper des B-Ab. Bei dem Sandwich-Test vermittelt wiederum das Probenantigen die Bindung zwischen dem Antikörper des B-Ab und dem Antikörper des Ab-M.

Bei dieser Ausführungsform bilden das trägerfixierte Streptavidin (oder Avidin) und das Biotin, welches mit anderen Reagenzien kovalent gebunden ist, ein sogenanntes "Capturing-System". Die Verwendung eines "Capturing-Systems" ermöglicht es, auf einfache Weise verschiedene Reagenzbestandteile an eine Festphase zu binden, welche einheitlich (mit einem einzigen Bindungsbestandteil, hier Streptavidin) vorbehandelt ist. Im Rahmen der vorliegenden Erfindung ist dies darüberhinaus in präzise lokalisierbarer Form möglich. Andererseits können auf eine einheitlich, beispielsweise mit Streptavidin vorbehandelte Tragschicht auch lösliche Kompartimente (nicht biotinylierte Reagenzien) appliziert werden, ohne daß deren Löslichkeitsverhalten wesentlich beeinflußt wird. Nähere Einzelheiten sind der EP-A-0 269 092 und der EP-A-0 344 578 zu entnehmen.

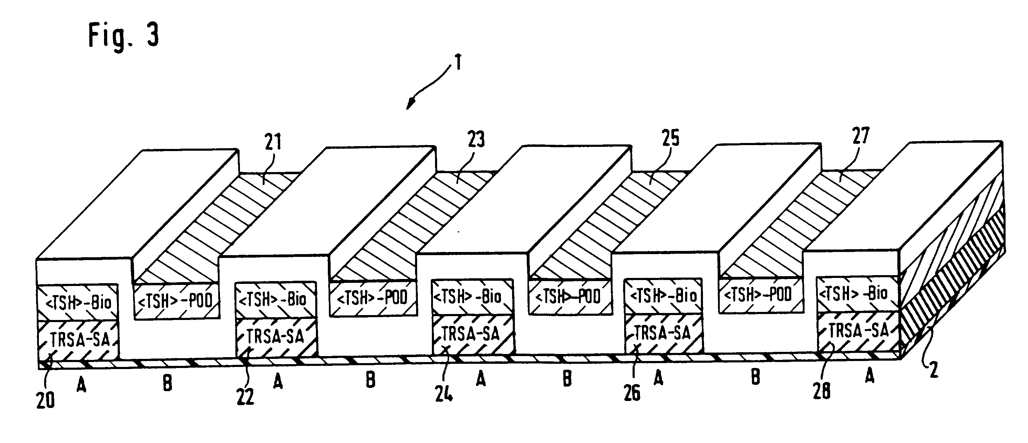

Figur 3 zeigt eine alternative Ausführungsform eines Testträgers mit drei Sätzen von Kompartimenten mit drei verschiedenen Bindungspartnern. Die Testzusammensetzung stimmt im wesentlichen mit der vorhergehenden Ausführungsform überein, wobei in diesem Fall jedoch der zweite Bindungspartner unmittelbar auf den ersten Bindungspartner beschichtet und somit an diesen gebunden ist. Die beiden gebundenen Bindungspartner werden deshalb als Bp(b)1 und Bp(b)2 bezeichnet. Sie befinden sich in Doppelkompartimenten 20,22,24,26,28 des Satzes A. Der dritte Bindungspartner ist ein freier Bindungspartner Bp(f) in den Kompartimenten 21,23,25,27 des Satzes B.

Die folgenden Beispiele dienen der weiteren Erläuterung der Erfindung. Soweit nicht anders vermerkt, bezeichnen alle %-Angaben Gewichts-Prozente.

### Beispiel 1:

Für die Applikation der Kompartimente wurde ein Tintenstrahldrucker SQ-2550 der Firma Epson verwendet. Anstelle des Tintenbehälters wurde ein separater Reagenzbehälter mit der jeweils zu dosierenden Reagenzlösung an das zum Druckkopf führende Schlauchsystem angeschlossen. Der Drucker wurde im Graphikmodus (Einzeldüsen-Ansteuerung) über einen Personal Computer betrieben.

Als Trägermaterial diente ein DIN A4-Zuschnitt aus Polystyrol-Folie mit 0,1 mm Dicke. Die Folie wurde vor ihrer Verwendung einer standardisierten Gamma-Bestrahlung unterzogen.

Charakteristika des Druckkopfes:
- 24 Düsen in zwei Reihen (halbzeilig versetzt) angeordnet
- Tropfendurchmesser ca. 90 µm
- kleinstes dosierbares Volumen (1 Tropfen): ca. 420 Pikoliter
- Druckdichte pro Druckschritt: 180x180 Dots/inch

Die Applikation der Kompartimente erfolgt in der in Verbindung mit Fig. 1 beschriebenen Verfahrensweise. Jedes der Reagenzkompartimente besteht aus 24 Einzeltropfen zu je ca. 420 Pikoliter. Die Abmessungen eines Kompartiments betragen in der Höhe ca. 3,2 mm und in der Breite ca. 0,06 bis 0,08 mm. Der Abstand von Mitte zu Mitte zwischen den Kompartimenten liegt bei ca. 0,26 mm.

Die Trennschicht auf Basis von Rinderserumalbumin (im folgenden "RSA-Trennschicht") wurde mit insgesamt vier Druckvorgängen mit einem Dotabstand von horizontal 0,14 mm und vertikal 0,14 mm aufgebracht.

Die Anordnung der Kompartimente in dem Reagenzbereich entsprach Fig. 2. Für die einzelnen Sätze von Kompartimenten wurden dabei folgende Lösungen eingesetzt.
a) Kompartiment-Satz A: Bp(b) (nämlich SA): Trägerfixiertes SA

| | | |
|---|---|---|
| Beschichtungslösung: | 0,25 mg/ml | TRSA-SA |
| | 40 mM | Natriumphosphat-Puffer(NaPP) pH 7,4 |
| | 5 Vol-% | iso-Propanol |

Mit TRSA-SA ist Thermo-Rinderserumalbumin-Streptavidin gemäß der EP-A-0 269 092 und der EP-A-0 344 578 bezeichnet. Diese Verbindung eignet sich in besonderem Maße zur adsorptiven Trägerfixierung von Streptavidin. Die pro Fläche aufgebrachte TRSA-SA-Menge war geringfügig kleiner als die adsorptive Bindekapazität der Trägerfolie. Auf diese Weise werden zusätzliche Prozeßschritte zu Sicherstellung einer quantitativen und stabilen Bindung an die Festphase vermieden.
b) Schutzschicht 5: RSA-Trennschicht

| | | |
|---|---|---|
| Beschichtungslösung: | 0,6 % | Rinderserumalbumin(RSA) |
| | 4,0 % | Saccharose |
| | 1,8 % | Natriumchlorid (NaCl) |
| | 5,0 Vol-% | iso-Propanol |

c) Kompartiment-Satz B: Bp(f)1 (nämlich B-Ag): Eluierbares Konjugat aus T3 und Biotin (T3-Biotin), hergestellt durch Kopplung von Biotin an N-butyloxycarbonyl-trijodthyronin (T3) über Pentamethylendiamin (Eur. J. Biochem. 131 (1980,333-338)).

| | | |
|---|---|---|
| Beschichtungslösung: | 200 ng/ml | T3-Biotin |
| | 120 mM | Natrium-Barbiturat |
| | 21,8 mM | NaPP pH 8,35 |
| | 0,04 % | 8-Anilino-1-naphtalinsulfonsäure (ANS) |
| | 0,02 % | 4-Aminoantipyrin |
| | 0,01 % | Merthiolat |
| | 0,25 % | Rinder-IgG (R-IgG) |
| | 1,0 % | Pluronic^{R} F68 |

d) Kompartiment-Satz C: Bp(f)2 (nämlich Ab-M): Eluierbares Konjugat aus einem polyklonalen gegen T3 gerichteten Antikörper und Peroxidase (PAK<T3>-POD).

| | | |
|---|---|---|
| Beschichtungslösung: | 22,2 U/ml | PAK<T3>-POD |
| | 120 mM | Natrium-Barbiturat |
| | 21,8 mM | NaPP pH 8,35 |
| | 0,04 % | ANS |
| | 0,02 % | 4-Aminoantipyrin |
| | 0,01 % | Merthiolat |
| | 0,25 % | R-IgG |
| | 1,0 % | Pluronic^{R} F68 |

Aus der beschichteten Polystyrolfolie wurden Testfelder von 3,2 mm Breite und 15 mm Länge geschnitten, auf denen die Kompartimente über die gesamte Breite senkrecht zur Längsrichtung verliefen. Die in einem derartigen Testfeld insgesamt in den jeweiligen Kompartimenten vorliegenden molaren Reagenzmengen betrugen:

| | |
|---|---|
| Im Kompartimentsatz C: | 0,05 femto Mol PAK<T3>-POD (=1,4µU) |
| Im Kompartimentsatz B: | 23,0 femto Mol T3-Biotin |
| Im Kompartimentsatz A: | ca 500,0 femto Mol Biotinbindestellen (durch TRSA-SA) |

Eine Analyse auf T3 wurde wie folgt durchgeführt:
- Es wurden jeweils 50 µl T3-haltige Probe auf ein Testfeld aufgebracht und für 2,5 h bei Raumtemperatur in einer Feuchtekammer inkubiert
- Das Testfeld wurde 4-mal mit 1 ml der für die Schutzschicht verwendeten RSA-Lösung gewaschen
- Es wurde ein Substrat für das Markierungsenzym, nämlich 50 µl Diaminobenzidin(DAB)-Färbereagenz (0,5 mg/ml DAB, 40 mM NaPP pH 7,4, 0,025 % Cobaltchlorid, 0,02 % Nickelsulfat, 0,01 % Wasserstoffperoxid) aufgebracht und eine Stunde bei Raumtemperatur in der Feuchtekammer inkubiert
- Danach wurde mit Wasser gewaschen.

Der Test basiert auf dem weiter oben erläuterten kompetitiven Prinzip: Das T3 konkurriert mit dem biotinylierten T3 um Bindungsstellen an dem PAK<T3>-POD-Konjugat.

Abhängig von der T3-Konzentration in der Probe ergab sich eine visuell erkennbare unterschiedlich intensive Anfärbung des Kompartiment-Satzes A. Über eine meßtechnische Erfassung des Kompartiments-Musters ist eine Kalibrierung und quantitative Auswertung möglich.

### Beispiel 2:

Aufbau- und Herstellungsverfahren entsprechend dem Beispiel 1 mit Ausnahme der Zusammensetzung der für die Kompartiment-Sätze B und C verwendeten Lösungen. Diese waren wie folgt zusammengesetzt:
a) Kompartiment-Satz B: Bp(f)l (nämlich B-Ab): Eluierbares Konjugat aus einem monoklonalen gegen TSH gerichteten Antikörper (ECACC 87122201) und Biotin (MAK<TSH>-Biotin). Die Biotinylierung des Antikörpers erfolgte gemäß JACS 100 (1978,3585-3590) durch Umsetzung mit N-hydroxysuccinimid-Biotin im Verhältnis 10:1.

| | | |
|---|---|---|
| Beschichtungslösung: | 80 mM | NaPP pH 7,4 |
| | 65 µg/ml | MAK<TSH>-Biotin |
| | 0,6 % | RSA |
| | 0,3 % | Rinder-IgG |

b) Kompartiment-Satz C: Bp(f)2 (nämlich Ab-E): Eluierbares Konjugat aus Peroxidase und einem monoklonalen gegen TSH gerichteten Antikörper (ECACC 87122202) (MAK<TSH>-POD).

| | | |
|---|---|---|
| Beschichtungslösung: | 50 mM | NaPP pH 7,4 |
| | 9 U/ml | MAK<TSH>-POD |

Der Testablauf entspricht dem heterogenen Ein-Schritt-Sandwich-Test mit Streptavidin-Biotin Capturing System zur Festphasenbindung des immunologischen Komplexes.

Es wurden wiederum Testfelder mit den gleichen Abmessungen wie bei Beispiel 1 hergestellt. Jedes Testfeld beinhaltet 18 Kompartimente des Satzes A und jeweils 9 Kompartimente der Sätze B und C.

Der Ablauf der Analyse entsprach Beispiel 1, wobei jedoch im ersten Schritt jeweils 100 µl TSH-haltige Proben aufgebracht wurden und eine Inkubation von 3 h bei RT in einer Feuchtekammer erfolgte.

Übereinstimmend mit Beispiel 1 ergab sich eine visuell deutlich erkennbare unterschiedlich intensive Anfärbung der auf dem Reagenzträger lokalisierten Kompartimente des Satzes A.

## Patentansprüche

1. Analyseelement zur Bestimmung eines Analyten in einer Probenflüssigkeit mittels einer spezifischen Bindungsreaktion bioaffiner Bindungspartner mit einer Tragschicht (2), welche in einem Reagenzbereich (4) einen mit einem Druckverfahren in einem definierten Muster applizierten trägerfixierten und somit festphasengebundenen Bindungspartner enthält,
**dadurch gekennzeichnet,** daß
das Muster mehrere mit einem Ink-Jet-Verfahren applizierte Sätze (A,B,C) von Kompartimenten (11-20) umfaßt, wobei
die Kompartimente eines ersten Satzes (A) fixierte Kompartimente sind, den trägerfixierten und somit festphasengebundenen Bindungspartner als ersten Bindungspartner enthalten, der zu einem zweiten Bindungspartner, welcher in der Probenflüssigkeit enthalten oder ein Reagenz ist, spezifisch bindungsfähig ist,
die Kompartimente eines zweiten Satzes (B) eluierbare Kompartimente sind, die einen zweiten Bindungspartner in löslicher Form enthalten, der mit dem ersten trägerfixierten Bindungspartner oder einem in der Probenflüssigkeit enthaltenen Bindungspartner spezifisch bindungsfähig ist und
die Kompartimente unterschiedlicher Sätze alternierend angeordnet sind, so daß die verschiedene Reagenzien enthaltenden Kompartimente eng benachbart, aber räumlich getrennt sind.

2. Analyseelement nach Anspruch 1, **dadurch gekennzeichnet,** daß die Konzentration des ersten trägerfixierten Bindungspartners in den fixierten Kompartimenten (A) geringer ist als die Bindungskapazität der Oberfläche, an die er fixiert ist.

3. Analyseelement nach einem der Ansprüche 1 oder 2, **dadurch gekennzeichnet,** daß die fixierten Kompartimente (A) von einer Schutzschicht (5a) auf Basis eines löslichen Proteins bedeckt sind.

4. Analyseelement nach einem der vorhergehenden Ansprüche, **dadurch gekennzeichnet,** daß unter den eluierbaren Kompartimenten (12,16) des zweiten Satzes (B) eine die Tragschicht (2) bedeckende Blockierungsschicht (5b) auf Basis eines löslichen Proteins angeordnet ist.

5. Analyseelement nach den Ansprüchen 3 und 4, **dadurch gekennzeichnet,** daß die Blockierungs- (5b) und die Schutzschichten (5a) ineinander übergehen, so daß sie eine durchgehende Trennschicht (5) zwischen den fixierten Kompartimenten des ersten Satzes (A) und den eluierbaren Kompartimenten des zweiten Satzes (B) bilden.

6. Analyseelement nach einem der vorhergehenden Ansprüche, dadurch gekennzeichnet, daß ein dritter Bindungspartner in einem weiteren Satz (C) von Kompartimenten (14,18) enthalten ist und der zweite Bindungspartner sowohl mit dem ersten als auch mit dem dritten Bindungspartner mit unterschiedlicher Spezifität spezifisch bindungsfähig ist.

7. Analyseelement nach Anspruch 6, **dadurch gekennzeichnet,** daß es ein Capturing-System, insbesondere auf Basis von Avidin oder Streptavidin und Biotin enthält.

8. Verfahren zur Herstellung eines Analyseelementes nach einem der vorhergehenden Ansprüche, **dadurch gekennzeichnet,** daß mit einem Ink-Jet-Düsenkopf eine Vielzahl von diskreten Reagenzflüssigkeitsquanten nacheinander auf die Reagenzfläche der Tragschicht ausgestoßen werden und der Düsenkopf und die Tragschicht relativ zu einander derartig bewegt werden, daß die von den Reagenzflüssigkeitsquanten erzeugten Dots einen Satz von Kompartimenten auf der Reagenzfläche bilden.

9. Verfahren nach Anspruch 8, **dadurch gekennzeichnet,** daß die Tragschicht aus Kunststoff besteht und vor der Applikation der Reagenzflüssigkeit gammabestrahlt wird.

10. Verfahren nach einem der Ansprüche 8 oder 9, **dadurch gekennzeichnet,** daß zur Erzeugung eines Kompartimentes in einem ersten Schritt die Reagenzflüssigkeitsquanten derartig appliziert werden, daß sie räumlich voneinander getrennte Dots auf der Reagenzfläche bilden und in mindestens einem weiteren zeitlich getrennten Schritt Reagenzflüssigkeitsquanten auf die Zwischenräume zwischen den ersten Dots derartig appliziert werden, daß ein durchgehendes Kompartiment gebildet wird.

11. Verfahren nach einem der Ansprüche 8 bis 10, **dadurch gekennzeichnet,** daß mehrere Kompartimente verschiedener Sätze gleichzeitig mit einem Mehrkanaldüsenkopf erzeugt werden.

## Claims

1. An analysis element for the determination of an analyte in a liquid sample by means of a specific binding reaction of bioreactive binding partners with a carrier layer (2) which contains, in a reagent domain (4), a binding partner applied in a defined pattern by a printing process, fixed to the carrier and thus solid-phase-bound,
**characterized in that**
the pattern comprises several sets (A,B,C) of compartments (11-20) applied with an ink-jet process, wherein
the compartments of a first set (A) are fixed compartments which contain the carrier-fixed and thus solid-phase-bound binding partner as a first binding partner, which is capable of binding specifically to a second binding partner which is contained in the sample liquid or is a reagent,
the compartments of a second set (B) are elutable compartments which contain a second binding partner in soluble form, which is capable of binding specifically with the first carrier-fixed binding partner or with a binding partner contained in the sample liquid, and
the compartments of different sets arranged alternately, so that the compartments containing different reagents are close together but spatially separated.

2. An analysis element according to Claim 1, characterized in that the concentration of the first solid-phase-bound partner in the fixed compartments (A) is lower than the binding capacity of the surface to which it is fixed.

3. An analysis element according to one of Claims 1 or 2, **characterized in that** the fixed compartments (A) are covered with a protective layer (5a) based on a soluble protein.

4. An analysis element according to any one of the preceding claims, **characterized in that** a blocking layer (5b) based on a soluble protein, covering the carrier layer (2), is arranged under the compartments (12, 16) of set (B).

5. An analysis element according to Claims 3 and 4, **characterized in that** the blocking layer (5b) and the protective layer (5a) are combined to form a continuous separating layer (5) between the fixed compartments of the first set (A) and the elutable compartments of the second set (B).

6. An analysis element according to any one of the preceding Claims, **characterized in that** a third binding partner is contained in another set (C) of compartments (14, 18) and the second binding partner is capable of binding specifically both to the first and to the third binding partners with different specificities.

7. An analysis element according to Claim 6, **characterized in that** it contains a capturing system preferably based on avidin or streptavidin and biotin.

8. A process for the manufacture of an analysis element according to any one of the preceding claims, **characterized in that** a large number of discrete quanta of liquid reagent are successively ejected from an ink-jet head on to the reagent domain of the carrier layer, and the jet head and the carrier layer are moved relative to one another in such a way that the dots produced by the quanta of liquid reagent form a set of compartments on the reagent domain.

9. A process according to Claim 8, **characterized in that** the carrier layer consists of plastic and is irradiated with gamma rays before application of the liquid reagent.

10. A process according to one of Claims 8 or 9, **characterized in that** to produce a compartment, the quanta of liquid reagent are applied, in a first step, so as to form dots on the reagent domain which are spatially separated from one another and, in at least one further step, at a different time, quanta of liquid reagent are applied to the spaces between the first dots so as to form a continuous compartment.

11. A process according to any one of Claims 8 to 10, **characterized in that** several compartments of different sets are produced simultaneously with a multichannel jet head.

## Revendications

1. Elément d'analyse pour la détermination d'un analyte dans un échantillon liquide, au moyen d'une réaction de liaison spécifique d'un partenaire de liaison présentant une affinité biologique, comportant une couche de support (2) dont une zone à réactifs (4) contient un partenaire de liaison qui est appliqué au moyen d'un procédé d'impression dans une matrice définie, fixé au support et de ce fait, lié à la phase solide, caractérisé par le fait que
la matrice comprend plusieurs jeux (A, B, C) de compartiments (11-20) produits à l'aide d'un procédé à jet d'encre, où
les compartiments d'un premier jeu (A) sont des compartiments fixés, qui contiennent le partenaire de liaison fixé au support et donc lié à la phase solide, en tant que premier partenaire de liaison capable de se lier spécifiquement à un second partenaire de liaison qui est contenu dans l'échantillon liquide ou qui est un réactif,
les compartiments d'un second jeu (B) sont des compartiments pouvant être élués, qui contiennent un second partenaire de liaison sous forme soluble, qui est capable de se lier spécifiquement au premier partenaire de liaison fixé au support ou à un partenaire de liaison contenu dans l'échantillon liquide, et
les compartiments des différents jeux sont disposés de façon alternée, de sorte que les compartiments contenant les différents réactifs sont très proches, mais séparés dans l'espace.

2. Elément d'analyse selon la revendication 1, caractérisé par le fait que la concentration du premier partenaire de liaison fixé au support dans les compartiments fixés (A) est plus faible que la capacité de liaison de la surface sur laquelle il est fixé.

3. Elément d'analyse selon l'une des revendications 1 ou 2, caractérisé par le fait que les compartiments fixés (A) sont recouverts d'une couche de protection (5a) à base d'une protéine soluble.

4. Elément d'analyse selon l'une quelconque des revendications précédentes, caractérisé par le fait que sous les compartiments pouvant être élués (12, 16) du second jeu (B), se trouve disposée une couche de blocage (5b) à base d'une protéine soluble, recouvrant la couche de support (2).

5. Elément d'analyse selon les revendications 3 et 4, caractérisé par le fait que les couches de blocage (5b) et de protection (5a) se suivent de façon à former une couche de séparation (5) continue entre les compartiments fixés du premier jeu (A) et les compartiments pouvant être élués du second jeu (B).

6. Elément d'analyse selon l'une quelconque des revendications précédentes, caractérisé par le fait qu'un troisième partenaire de liaison est contenu dans un autre jeu (C) de compartiments (14, 18) et que le second partenaire de liaison est capable de se lier spécifiquement au premier partenaire de liaison aussi bien qu'au troisième partenaire de liaison, avec des spécificités différentes.

7. Elément d'analyse selon la revendication 6, caractérisé par le fait qu'il contient un système de capture, en particulier à base d'avidine ou de streptavidine et de biotine.

8. Procédé de préparation d'un élément d'analyse selon l'une quelconque des revendications précédentes, caractérisé par le fait qu'au moyen d'une tête d'injecteur à jet d'encre, on dépose une multitude de quanta discrets de réactif liquide, les uns après les autres, sur la surface à réactifs de la couche de support et que la tête d'injecteur et la couche de support se déplacent l'une par rapport à l'autre de façon que les points formés par les quantités de réactif liquide forment un jeu de compartiments sur la surface à réactifs.

9. Procédé selon la revendication 8, caractérisé par le fait que la couche de support est en matière plastique et qu'avant l'application du réactif liquide, elle subit une irradiation par les rayons gamma.

10. Procédé selon la revendication 8 ou 9, caractérisé par le fait que pour obtenir un compartiment, dans une première étape on applique les quanta de réactif liquide de façon qu'ils forment, sur la surface à réactifs, des points séparés entre eux dans l'espace et que dans au moins une autre étape séparée dans le temps, on applique des quanta de réactif liquide dans les interstices entres les premiers points de façon à former un compartiment continu.

11. Procédé selon l'une quelconque des revendications 8 à 10, caractérisé par le fait que plusieurs compartiments de différents jeux sont obtenus simultanément avec une tête d'injecteur à plusieurs canaux.
